# NEW EUROPEAN PATENT SPECIFICATION

(11) **EP 3 471 807 B2**
(45) Date of publication and mention of the opposition decision: **07.10.2026**
(45) Mention of the grant of the patent: 01.07.2020
(21) Application number: 17727924.7
(22) Date of filing: 08.06.2017
(51) Int. Cl.: A61M 15/06, A24F 47/00, A61M 11/04

(54) **VAPORISER ASSEMBLY FOR AN AEROSOL-GENERATING SYSTEM**
VERDAMPFERANORDNUNG FÜR EIN AEROSOLERZEUGUNGSSYSTEM
ENSEMBLE DE VAPORISATEUR POUR SYSTÈME DE GÉNÉRATION D'AÉROSOL

(30) Priority: 20.06.2016 EP 16175303
(43) Date of publication of application: 24.04.2019
(73) Proprietor: Philip Morris Products S.A., 2000 Neuchâtel (CH)
(72) Inventor: DUC, Fabien, 1227 Carouge (CH)
(74) Representative: Reddie & Grose LLP
(86) International application number: PCT/EP2017/064045
(87) International publication number: WO 2017/220340

(56) References cited:
- WO-A1-2013/083638
- WO-A1-2015/114325
- WO-A2-2015/117705
- CN-A- 105 394 816
- GB-A- 2 504 076
- US-A1- 2007 107 879
- US-A1- 2013 307 177
- US-A1- 2014 261 487
- US-A1- 2014 299 125
- US-A1- 2015 196 060
- US-A1- 2015 359 262

## Description

The present invention relates to an aerosol-generating system with a vaporiser assembly.

Handheld electrically operated aerosol-generating systems are known that consist of a device portion comprising a battery and control electronics and a separate cartridge comprising a supply of liquid aerosol-forming substrate held in a liquid storage portion and an electrically operated vaporiser or heater element. The liquid storage portion may comprise a capillary material in which the liquid aerosol-forming substrate is absorbed. The capillary material is in contact with the heater element and ensures that the liquid is conveyed to the heater element, thereby allowing the creation of vapor. The vapor subsequently cools to form an aerosol. It is known, for example from WO 2015/117702 A1, that the capillary material can have a fibrous or spongy structure. The capillary material may be a porous material conveying the liquid from the liquid storage portion to the heater element. The capillary material and the heater element are provided, together with the liquid storage portion, in the cartridge. The cartridge is provided as a single-use cartridge, which is disposed once the liquid aerosol-forming substrate held in the liquid storage portion is depleted. The capillary material and the heater element are therefore disposed together with the cartridge and new capillary material and a new heater element are required for each new cartridge. Furthermore, unwanted burning residues can develop on a surface of the capillary material during use.

US 2007/107879 A1 discloses a liquid evaporator comprising a heater and an evaporator tube. The evaporator tube has a first porous element which may comprise porous sintered glass. The first porous element has an area in contact with a liquid reservoir. A second porous element, in contact with the first porous element, is heated by the heater. The first porous element may have a relatively coarse pore structure and the second porous element may have a relatively fine porosity.

It is desirable to provide a vaporiser assembly which is easy to clean and re-usable, decreasing the cost of the consumable. Also, it is desirable to provide a vaporiser assembly having an increased heat resistance and which avoids or at least reduces the risk of emitting undesirable products when operated at elevated temperatures.

According to an aspect of the present disclosure there is provided a vaporiser assembly for an aerosol-generating system, comprising a capillary element which comprises porous glass. The capillary element has a first end and a second end. The vaporiser assembly further comprises a heater element. The first end of the capillary element is configured to be fluidly connected to a liquid storage portion and the heater element is provided at the second end of the capillary element. The pore size of the capillary element is configured to allow a liquid aerosol-forming substrate from the liquid storage portion to be conveyed from the first end of the capillary element to the second end of the capillary element by capillary action. The average pore size of the capillary element varies from large pores at the first end of the capillary element to small pores at the second end of the capillary element such that a pore size gradient from the first end of the capillary element to the second end of the capillary element is provided.

Due to the fact that the capillary element is made from glass, the heater element may be provided directly on the capillary element. In this regard, an advantage of the porous glass of the capillary element is that glass has an increased heat resistance. The capillary element is consequently not damaged or harmed by the increased temperature of the heating element during heating, even if the heater element is provided directly on the capillary element or in the near vicinity of the capillary element.

An increased heat resistance of the capillary element also leads to the effect that during heating of the liquid aerosol-forming substrate by the heater element, the risk of emitting undesirable products is reduced.

Also, since the capillary element is made from porous glass, the capillary element may be easily cleaned. The capillary element may be manually cleaned by a user when the user changes the replaceable liquid storage portion. Also or additionally, the capillary element may be cleaned during the insertion of the capillary element into a new liquid storage portion.

By providing the porous glass in the capillary element, the improved cleaning and heat resistance synergistically improve the reusability of the vaporiser assembly. Due to the improved heat resistance unwanted residues on the capillary element and thus undesirable products are avoided or reduce during heating. The heater element may also be provided directly on the capillary element or in the near vicinity of the capillary element. At the same time, unwanted residues on the capillary element may be easily cleaned.

Furthermore, glass is a very stable material, which does not degrade with temperature. Multiple replaceable liquid storage portions may therefore be used before the capillary element must be replaced.

Liquid storage portions may be used without the need to provide a new capillary element and a new heater element each time the liquid storage portion is replaced. The capillary element as well as the heater element is useable with multiple replaceable liquid storage portions. Therefore, the costs for the replaceable liquid storage portion are decreased.

The capillary element may have a cylindrical form or a different form suited to be inserted into a replaceable liquid storage portion. The capillary element has a first surface at the first end and a second surface at the second end. The heater element is provided on the second end surface of the capillary element. The first surface and the second surface may have a round or ellipsoidal shape. Also, the first and second surface may have a rectangular shape or a polygonal shape. Furthermore, the first surface and the second surface may be substantially flat or curved. A side surface is provided at the circumference of the capillary element between the first end and the second end. The first and second surface may have a diameter of between 1 millimeter and 15 millimeters, preferably between 2 millimeter and 10 millimeters, more preferably between 3 millimeter and 7 millimeters, more preferably between 4 millimeter and 6 millimeters, most preferably around 5 millimeters. The surface area of the first and second surface may be smaller than 60 square millimeters, preferably smaller than 50 square millimeters, preferably smaller than 40 square millimeters, and most preferably around 30 square millimeters. The length of the capillary element may be between 1 millimeter and 7,5 centimeters, preferably between 5 millimeters and 3 centimeters, and more preferably around 1 centimeter. The liquid capacity of the capillary element is such that it can hold enough liquid aerosol-forming substrate for 30 to 40 puffs of more, preferably around 32 puffs. A 3 second puff may include between 1 milligram and 4 milligrams of liquid, preferably between 3 milligrams and 4 milligrams of liquid. The capacity of the capillary element may be between 30 milligrams and 160 milligrams, preferably between 60 milligrams to 150 milligrams, more preferably between 90 milligrams to 140 milligrams, most preferably around 130 milligrams.

The capillary element is made from porous glass. The glass has an internal structure which allows liquids to be conveyed from the first end of the capillary element to the second end of the capillary element. In more detail, the porous glass comprises pores which enable liquid to travel through the capillary element.

In this regard, the pores, which are provided in the capillary element, enable the effect of intermolecular forces between the liquid aerosol-forming substrate and the surrounding glass material of the capillary element. The size and preferably the diameter of the pores is configured such that the combination of surface tension and adhesive forces between the liquid aerosol-forming substrate and the surrounding glass material of the capillary element leads to the conveying of the liquid through the capillary element.

The term "porous" should be understood in a broad meaning. The pores of the capillary element are interconnected and may have a fibrous structure. The capillary element preferably comprises a bundle of capillaries. For example, the capillary element is manufactured by assembling and compressing glass particles similar to the manufacturing of ceramics. The size of the pores, which are generated during this process, depends on the applied force during the compression. The pore size may vary along the cylinder. The pores may be generally aligned to convey the liquid aerosol-forming substrate to the heater element. The structure of the capillary element forms a plurality of small pores, through which the liquid may be transported by capillary action. The capillary element may have any suitable capillarity and porosity so as to be used with different liquid physical properties. The liquid has physical properties, including but not limited to viscosity, surface tension, density, thermal conductivity, boiling point and vapor pressure, which allow the liquid to be transported through the capillary element by capillary action.

The capillary element may comprise multiple materials, wherein one of these materials is the porous glass. The capillary element may also be entirely made of the porous glass. Also, multiple capillary elements could be provided next to each other, wherein one or more of the above capillary elements could be combined.

The capillary element may have a form that when the capillary element is inserted into a liquid storage portion, liquid present in the liquid storage portion cannot flow past the outer circumference of the capillary element. Consequently, liquid can only be conveyed out of the liquid storage portion through the capillary element. A press-fit may be provided between the capillary element and the liquid storage portion, when the liquid storage portion is connected to the capillary element, such that liquid from the liquid storage portion may only flow out of the liquid storage portion through the capillary element. Liquid is prevented from flowing through the side surface of the capillary element by the liquid storage portion. In more detail, the press-fit between the capillary element and the liquid storage portion prevents that the liquid flows through the side surface of the capillary element.

Alternatively, the pores, which are provided in the capillary element, are provided in a longitudinal direction between the first and second end of the capillary element such that liquid may only flow through the capillary element from the first end of the capillary element to the second end of the capillary element. The capillary element may comprise a fluid impermeable outer surface such as a fluid impermeable coating. The fluid impermeable coating may be applied to the outer surface of the capillary element to prevent leakage. Alternatively, the capillary element may be inserted into a fluid impermeable tube such as a glass tube. Liquid cannot flow through the capillary element at the side surface of the capillary element, since the pores are not provided at the side surface of the capillary element.

As a further alternative, the pores in the capillary element are - at a side surface of the capillary element - provided with a size which prevents liquid to leak out of the side surface of the capillary element. In other words, the diameter or size of the pores, which are provided at the side surface of the capillary element, is so small that liquid cannot flow through these pores at the side surface of the capillary element.

In each case, liquid may enter the capillary element at the first end of the capillary element and is conveyed through the capillary element in the direction of the second end of the capillary element.

The average pore size of the pores which are provided in the porous glass varies from large pores at the first end of the capillary element to small pores at the second end of the capillary element. In that way, a pore size gradient is provided from the first end of the capillary element to the second end of the capillary element.

Smaller pores create a larger capillary force or action. Consequently, providing smaller pores at the second end of the capillary element ensures that the liquid aerosol-forming substrate from the liquid storage portion is drawn from the first end towards the second end of the capillary element. The pore size is configured to optimize the flow rate. Smaller pores also prevent liquid to be leaked out of the capillary element. Only vapor may flow through the small pores to enable a subsequent formation of an aerosol. The pore size of the small pores is, according to the invention, between 2 and 8 microns or about 4 microns.

The average pore size of the pores at the first end of the capillary element is larger than the average pore size of the pores at the second end of the capillary element. The average pore size is an average pore size for a region of the capillary element. In this way it can be seen that the liquid aerosol-forming substrate is conveyed more efficiently to the heater element. The pore size of the large pores is, according to the invention, between 5 and 500 microns or between 10 and 250 microns or between 15 and 100 microns or between 20 and 50 microns.

By providing a pore size gradient, preferably a linear gradient, in the capillary element, the effect is achieved that an aerosol-forming substrate in the form of a liquid may be efficiently and in relatively large amounts conveyed from the liquid storage portion at the first end of the capillary element to the second end of the capillary element, which is adjacent to the heater element. The liquid may then be vaporised by the heater element next to the second end of the capillary element.

The heater element is provided at the second end of the capillary element such that liquid which is conveyed through the capillary element from the first end to the second end may be vaporised by the heater element. The heater element may be provided directly on the second end of the capillary element so that the heater element directly contacts the second end of the capillary element. Alternatively, the heater element may be provided in the close proximity of the second end of the capillary element. In the latter case, the heater element is provided to heat the second end of the capillary element. The heater element may be provided at the circumference of the capillary element adjacent to the second end of the capillary element.

By providing the heater element at the circumference of the capillary element adjacent to the second end of the capillary element, a compact vaporiser assembly, comprising the capillary element and the heater element may be provided. Also, an efficient vaporisation of the liquid, which is conveyed from the first end of the capillary element to the second end of the capillary element, can be provided. By providing the heater element at the circumference of the capillary element adjacent to the second end of the capillary element, the capillary element can be easily cleaned due to the second end surface of the capillary element not being blocked by the heating element.

In some embodiments, the heater element is an electric resistance heater. The heater element comprises an electrically conductive material. The electrically conductive material may be heated by an electric current flowing through the electrically conductive material. The electrically conductive material may be provided on an electrically insulating substrate of the heater element.

The heater element may also comprise a glass material such that the capillary element and the heater element each comprise glass material. The electrically conductive material of the heater element may be provided in or on the heater element.

The electrical resistance of the heater element should be provided so that a sufficient heating of the aerosol-forming substrate at the second end surface of the capillary element is provided. In this regard, the electrical resistance of the electrically conductive material of the heater element may be between 2 ohm and 5 ohm, preferably between 3 ohm and 4 ohm, and most preferably around 3.5 ohm.

In some embodiments, the heater element may be provided as a metallic coating or thin film or a mesh heater or a coil. When the heater element is provided as a metallic coating, the heater element may be provided directly on the second end surface of the capillary element. In more detail, the second end surface of the capillary element may be provided with an electrically conductive coating, which may be heated to vaporise liquid on the second end surface of the capillary element.

The heater element may also be provided as a mesh heater, which comprises multiple conductive filaments. This allows a greater area of the heater element to be in contact with a liquid being vaporised. The electrically conductive filaments may be substantially flat.

The heater element may be provided as a heater coil made from electrically conductive wire. A coil may be wound around the capillary element and is beneficial in case that the heater element is provided at the circumference of the capillary element adjacent to the second end of the capillary element.

Also provided is a vaporiser assembly for an aerosol-generating system, comprising a capillary element which comprises porous glass. The capillary element has a first end and a second end. The vaporiser assembly further comprises a heater element. The first end of the capillary element is configured to be fluidly connected to a liquid storage portion and the heater element is provided at the second end of the capillary element. The pore size of the capillary element is configured to allow a liquid aerosol-forming substrate from the liquid storage portion to be conveyed from the first end of the capillary element to the second end of the capillary element by capillary action.

According to the present invention, an aerosol-generating system is provided, which comprises a main body. The main body comprises a housing, a power supply, electric circuitry and a vaporiser assembly as described in detail above.

The aerosol-generating system further comprises a replaceable liquid storage portion. The liquid storage portion is detachably connectable to the main body. When the liquid storage portion is attached to the main body, the first end of the capillary element of the vaporiser assembly is inserted into the liquid storage portion, such that the capillary element comes into fluid communication with the liquid aerosol-forming substrate stored in the liquid storage portion.

The liquid storage portion may comprise a further capillary element. The further capillary element may be provided in the liquid storage portion. In this case, the glass capillary element of the vaporiser may be thin to prevent the heater element from burning the further capillary element, which is provided in the liquid storage portion. The glass capillary element may have a thickness of at least 1 millimeter, preferably at least 2 millimeters and more preferably at least 3 millimeters. The glass capillary element of the vaporiser assembly may still comprise small pores such that liquid aerosol-forming substrate does not leak through the capillary element, but vapor may flow through the capillary element for aerosol formation. The further capillary element may be provided from a conventional porous material with a spongy or fibrous structure. The glass capillary element of the vaporiser assembly may be reusable, whereas the further capillary element may be disposed together with the liquid storage portion. Thus, the advantageous characteristics of glass can be utilized, while only a thin glass capillary element is necessary in the vaporiser.

The power supply may be electrically connected to the heater element of the vaporiser assembly to enable heating of the heater element. The electric circuitry controls the flow of electric current from the power supply to the heater element. When the aerosol-generating device is actuated by a user, the electric circuitry enables electric current to flow from the power supply to the heater element of the vaporiser assembly, thereby vaporising an aerosol-forming substrate from a liquid storage portion and creating an aerosol. A sensor such as a flow sensor may be provided to detect that a user draws on the system.

In some embodiments, a sealing foil may be provided on an opening of the liquid storage portion. During or before insertion of the capillary element into the liquid storage portion, the sealing foil is preferably removed. The aerosol-generating system preferably further comprises a sealing membrane, which is disposed beneath the sealing foil. When the sealing foil is removed and the capillary element is inserted into the liquid storage portion, the sealing membrane may be ruptured and pressed between the circumference of the capillary element and the replaceable liquid storage portion. The sealing membrane is provided to prevent undesired leakage of the liquid aerosol-forming substrate out of the liquid storage portion. In other words, liquid aerosol-forming substrate may flow through the capillary element but not past the outer circumference of the capillary element, when the capillary element is inserted into the liquid storage portion.

According to a further aspect of the present disclosure, a method for manufacturing a vaporiser assembly for an aerosol generating system is provided. The method comprises the steps of providing a capillary element made from porous glass, the capillary element having a first end and a second end, and providing a heater element. The first end of the capillary element is configured to be fluidly connected to a liquid storage portion, wherein the heater element is provided at the second end of the capillary element. The pore size of the capillary element is provided to allow a liquid aerosol-forming substrate from the liquid storage portion to be conveyed from the first end of the capillary element to the second end of the capillary element by capillary action. The average pore size of the capillary element varies from large pores at the first end of the capillary element to small pores at the second end of the capillary element such that a pore size gradient from the first end of the capillary element to the second end of the capillary element is provided.

In some embodiments, the capillary element is manufactured by a phase separation process, a sintering process or a sol-gel process. These processes enable that the capillary element is provided with pores, which in turn enable that a liquid aerosol-forming substrate is conveyed through the capillary element. Furthermore, these processes enable that the capillary element is produced with pores of different size and with a pore size gradient from a first end of the capillary element to a second end of the capillary element.

Features described in relation to one aspect may equally be applied to other aspects of the invention.

Embodiments of the invention will now be described, by way of example only, with reference to the accompanying drawings, in which:
Figure 1 is a sectional view of an embodiment of a vaporiser assembly; and
Figure 2 is a sectional view of an embodiment of an aerosol-generating system.

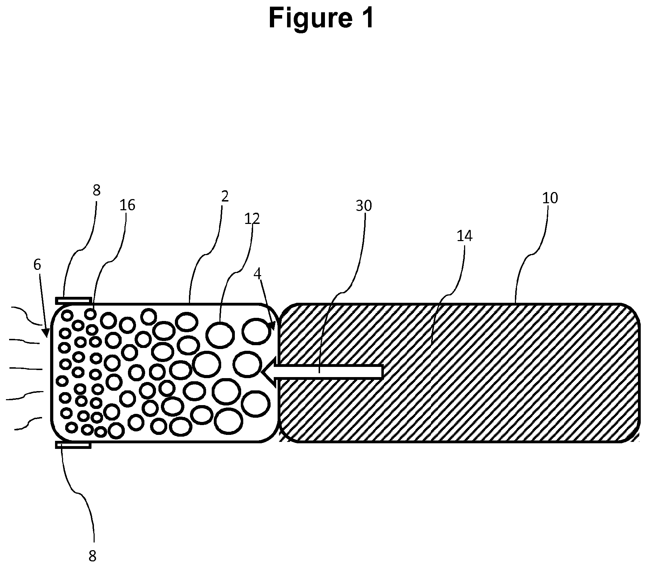

Figure 1 shows a vaporiser assembly.

The vaporiser assembly comprises a capillary element 2 made from porous glass as depicted in the left part of Figure 1. The capillary element 2 comprises pores of preferably varying size. The capillary element 2 has a first end 4 and a second end 6.

The porous glass of the vaporiser assembly is provided adjacent to a heater element 8. The heater element 8 as depicted in Figure 1 is disposed at the circumference of the porous glass adjacent to the second end 6 of the porous glass. The first end 4 of the capillary element 2 faces a liquid storage portion 10.

At the first end 4 of the capillary element 2, large pores 12 with an average pore size of around 25 microns are provided as depicted in Figure 1. The large pores 12 enable a liquid aerosol-forming substrate 14 to be conveyed from the liquid storage portion 10 in the direction of the second end 6 of the capillary element 2 through the capillary element 2. At the second end 6 of the capillary element 2, small pores 16 with an average pore size of around 4 microns are provided, preventing liquid to leak out of the second end 6 while enabling a flow of vapor through the second end 6.

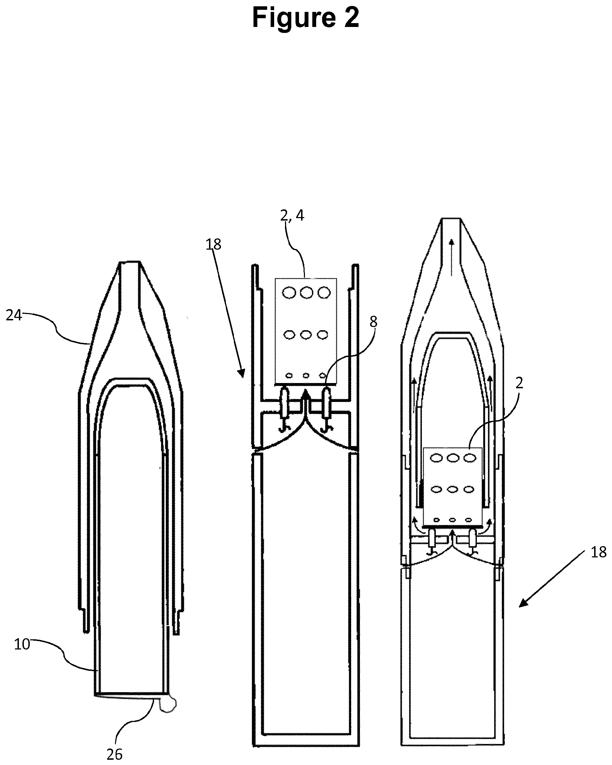

Figure 2 shows an aerosol-generating system according to the present invention. Depicted in Figure 2 is a main body 18 comprising the capillary element 2 of the vaporiser assembly, the heater element 8 as well as electrical circuitry and a power supply (not depicted in Figure 2). The main body further comprises air inlets 20 and an air outlet 22. When a user sucks on a mouthpiece 24, ambient air is drawn through the air inlets 20 past the vaporiser assembly towards the air outlet 22. The electrical circuitry controls the flow of electric current from the power supply to the heater element 8 for heating the heater element 8. A flow sensor may detect - due to a negative pressure in the airflow - when a user draws on the mouthpiece 24. Then, the electrical circuitry controls a flow of electric current from the power supply through the heater element. Consequently, the liquid aerosol forming substrate 14 is vaporised by the heater element 8, thereby creating an aerosol which is subsequently inhaled by the user.

The liquid storage portion 10 is provided with a sealing foil 26 which is provided on the liquid storage portion 10. The sealing foil 26 prevents that aerosol-forming substrate 14 leaks out of the liquid storage portion 10. The sealing foil 26 is removed before the liquid storage portion 10 is attached to the main body 18. Beneath the sealing foil 26, a sealing membrane 28 is provided covering the liquid storage portion 10. The sealing membrane 28 is provided for sealing the outer circumference of the capillary element 2 when the capillary element 2 is inserted into the liquid storage portion 10 as described in the following.

When the liquid storage portion 10 is attached to the main body 18, the capillary element 2 of the vaporiser assembly is inserted into the liquid storage portion 10. The capillary element 2 is inserted into the liquid storage portion 10 such that the first end 4 of the capillary element 2 is inserted first into the liquid storage portion 10. During insertion of the capillary element 2 into the liquid storage portion 10, the sealing membrane 28 is ruptured and pressed against the inner wall of the liquid storage portion 10. The sealing membrane 28 may be provided with a predetermined breaking area or a predetermined breaking point or an area with small damage to localize and facilitate the rupture. Thus, the liquid aerosol-forming substrate 14 can only flow through the capillary element 2, while the outer circumference of the capillary element 2 is sealed by the sealing membrane 28.

The liquid-aerosol forming substrate 14 can then be conveyed from the inside of the liquid storage portion 10 through the first end 4 towards the second end 6 of the capillary element 2 of the vaporiser assembly in the direction 30 to the heater element 8 of the vaporiser assembly by capillary action.

The exemplary embodiments described above illustrate but are not limiting. The scope of the invention is as defined by the appended claims.

### Reference signs

- 2: capillary element
- 4: first end of the capillary element, first end surface of the capillary element
- 6: second end of the capillary element, second end surface of the capillary element
- 8: heater element
- 10: liquid storage portion
- 12: large pores
- 14: liquid aerosol-forming substrate
- 16: small pores
- 18: main body
- 20: air inlets
- 22: air outlet
- 24: mouthpiece
- 26: sealing foil
- 28: sealing membrane
- 30: direction in which the liquid aerosol-forming substrate is conveyed

## Claims

1. An aerosol-generating system, comprising:
a main body (18), the main body (18) comprising a housing, a power supply, electric circuitry and a vaporiser assembly, the vaporiser assembly comprising:
a capillary element (2) made from porous glass, the capillary element (2) having a first end (4) and a second end (6); and
a heater element (8),
wherein the first end of the capillary element (4) is configured to be fluidly connected to a liquid storage portion (10),
wherein the heater element (8) is provided at the second end of the capillary element (6), and
wherein the pore size of the capillary element (2) is configured to allow a liquid aerosol-forming substrate (14) from the liquid storage portion (10) to be conveyed from the first end of the capillary element (4) to the second end of the capillary element (6) by capillary action,
wherein the average pore size of the capillary element (2) varies from large pores (12) at the first end of the capillary element (4) to small pores (16) at the second end of the capillary element (6) such that a pore size gradient from the first end of the capillary element (4) to the second end of the capillary element (6) is provided,
wherein the average pore size of the small pores (16) is between 2 and 8 microns, and
wherein the average pore size of the large pores (12) is between 5 and 500 microns,
wherein the aerosol-generating system further comprises a replaceable liquid storage portion (10), detachably connectable to the main body (18), wherein the first end of the capillary element (4) of the vaporiser assembly is inserted into the liquid storage portion (10) when the liquid storage portion (10) is attached to the main body (18), such that the capillary element (2) comes into fluid communication with the liquid aerosol-forming substrate (14) stored in the liquid storage portion (10).

2. The aerosol-generating system according to claim 1, wherein the capillary element (2) has a generally cylindrical shape with a first surface at the first end and a second surface at the second end, wherein the heater element (8) is provided on the second end surface of the capillary element.

3. The aerosol-generating system according to claim 1 or claim 2, wherein the heater element (8) is provided at the circumference of the capillary element (2) adjacent to the second end of the capillary element (6).

4. The aerosol-generating system according to any one of the preceding claims, wherein the heater element (8) is an electric resistance heater.

5. The aerosol-generating system according to any one of the preceding claims, wherein the heater element (8) is provided as a metallic coating, a mesh heater or a coil.

6. The aerosol-generating system according to any one of the preceding claims, wherein the pore size gradient of the capillary element (2) is a linear pore size gradient.

7. The aerosol-generating system according to any one of the preceding claims, wherein the pore size of the small pores (16) is 4 microns.

8. The aerosol-generating system according to any one of the preceding claims, wherein the pore size of the large pores (12) is between 10 and 250 microns, preferably between 15 and 100 microns, and most preferably between 20 and 50 microns.

9. The aerosol-generating system according to any one of the preceding claims, further comprising a sealing element to be disposed between the circumference of the capillary element (2) and the replaceable liquid storage portion (10), in order to prevent undesired leakage of the liquid aerosol-forming substrate (14) out of the liquid storage portion (10).

## Patentansprüche

1. Aerosolerzeugungssystem, umfassend:
einen Hauptkörper (18), wobei der Hauptkörper (18) ein Gehäuse, eine Energieversorgung, eine elektrische Schaltung und eine Zerstäuberbaugruppe umfasst, wobei die Zerstäuberbaugruppe Folgendes umfasst:
ein Kapillarelement (2), das aus porösem Glas hergestellt ist, wobei das Kapillarelement (2) ein erstes Ende (4) und ein zweites Ende (6) aufweist; und
ein Heizelement (8),
wobei das erste Ende des Kapillarelements (4) derart ausgelegt ist, dass es mit einem Flüssigkeitsspeicherteil (10) in Fluidverbindung steht,
wobei das Heizelement (8) an dem zweiten Ende des Kapillarelements (6) vorgesehen ist, und
wobei die Porengröße des Kapillarelements (2) so ausgelegt ist, dass sie ermöglicht, dass ein flüssiges aerosolbildendes Substrat (14) aus dem Flüssigkeitsspeicherteil (10) durch Kapillarwirkung von dem ersten Ende des Kapillarelements (4) zu dem zweiten Ende des Kapillarelements (6) transportiert wird,
wobei die durchschnittliche Porengröße des Kapillarelements (2) von großen Poren (12) am ersten Ende des Kapillarelements (4) zu kleinen Poren (16) am zweiten Ende des Kapillarelements (6) variiert, sodass ein Porengrößengradient von dem ersten Ende des Kapillarelements (4) zu dem zweiten Ende des Kapillarelements (6) vorgesehen ist,
wobei die durchschnittliche Porengröße der kleinen Poren (16) zwischen 2 und 8 Mikrometer liegt, und
wobei die durchschnittliche Porengröße der großen Poren (12) zwischen 5 und 500 Mikrometer liegt,
wobei das Aerosolerzeugungssystem ferner einen auswechselbaren Flüssigkeitsspeicherteil (10) umfasst, der mit dem Hauptkörper (18) lösbar verbindbar ist, wobei das erste Ende des Kapillarelements (4) der Zerstäuberbaugruppe in den Flüssigkeitsspeicherteil (10) eingeführt wird, wenn der Flüssigkeitsspeicherteil (10) am Hauptkörper (18) angebracht wird, sodass das Kapillarelement (2) in Fluidverbindung mit dem flüssigen aerosolbildenden Substrat (14) kommt, das in dem Flüssigkeitsspeicherteil (10) gespeichert ist.

2. Aerosolerzeugungssystem nach Anspruch 1, wobei das Kapillarelement (2) eine im Allgemeinen zylindrische Form mit einer ersten Fläche am ersten Ende und einer zweiten Fläche am zweiten Ende aufweist, wobei das Heizelement (8) auf der zweiten Endfläche des Kapillarelements vorgesehen ist.

3. Aerosolerzeugungssystem nach Anspruch 1 oder Anspruch 2, wobei das Heizelement (8) am Umfang des Kapillarelements (2) angrenzend an das zweite Ende des Kapillarelements (6) vorgesehen ist.

4. Aerosolerzeugungssystem nach einem der vorhergehenden Ansprüche, wobei das Heizelement (8) eine elektrische Widerstandsheizvorrichtung ist.

5. Aerosolerzeugungssystem nach einem der vorhergehenden Ansprüche, wobei das Heizelement (8) als eine metallische Beschichtung, eine Netz-Heizvorrichtung oder eine Spule vorgesehen ist.

6. Aerosolerzeugungssystem nach einem der vorhergehenden Ansprüche, wobei der Porengrößengradient des Kapillarelements (2) ein linearer Porengrößengradient ist.

7. Aerosolerzeugungssystem nach einem der vorhergehenden Ansprüche, wobei die Porengröße der kleinen Poren (16) 4 Mikrometer beträgt.

8. Aerosolerzeugungssystem nach einem der vorhergehenden Ansprüche, wobei die Porengröße der großen Poren (12) zwischen 10 und 250 Mikrometer, bevorzugt zwischen 15 und 100 Mikrometer und am meisten bevorzugt zwischen 20 und 50 Mikrometer, liegt.

9. Aerosolerzeugungssystem nach einem der vorhergehenden Ansprüche, das ferner ein Dichtungselement umfasst, das zwischen dem Umfang des Kapillarelements (2) und dem auswechselbaren Flüssigkeitsspeicherteil (10) anzuordnen ist, um ein unerwünschtes Austreten des flüssigen aerosolbildenden Substrats (14) aus dem Flüssigkeitsspeicherteil (10) zu verhindern.

## Revendications

1. Système de génération d'aérosol, comprenant :
un corps principal (18), le corps principal (18) comprenant un logement, une alimentation électrique, une circuiterie électrique et un ensemble vaporisateur, l'ensemble vaporisateur comprenant :
un élément capillaire (2) composé de verre poreux, l'élément capillaire (2) ayant une première extrémité (4) et une deuxième extrémité (6) ; et
un élément de chauffage (8),
dans lequel la première extrémité de l'élément capillaire (4) est configurée pour être raccordée fluidiquement à une portion de stockage liquide (10),
dans lequel l'élément de chauffage (8) est prévu au niveau de la deuxième extrémité de l'élément capillaire (6), et
dans lequel la taille des pores de l'élément capillaire (2) est configurée pour permettre à un substrat formant aérosol liquide (14) provenant de la portion de stockage liquide (10) d'être acheminé par action capillaire depuis la première extrémité de l'élément capillaire (4) jusqu'à la deuxième extrémité de l'élément capillaire (6),
dans lequel la taille moyenne des pores de l'élément capillaire (2) varie de grands pores (12) au niveau de la première extrémité de l'élément capillaire (4) à de petits pores (16) au niveau de la deuxième extrémité de l'élément capillaire (6), de telle sorte qu'il est fourni un gradient de taille de pores depuis la première extrémité de l'élément capillaire (4) jusqu'à la deuxième extrémité de l'élément capillaire (6),
dans lequel la taille moyenne des pores des petits pores (16) est entre 2 et 8 microns, et
dans lequel la taille moyenne des grands pores (12) est entre 5 et 500 microns,
dans lequel le système de génération d'aérosol comprend en outre une portion de stockage liquide (10) remplaçable, raccordable de manière détachable au corps principal (18), dans lequel la première extrémité de l'élément capillaire (4) de l'ensemble vaporisateur est insérée jusque dans la portion de stockage liquide (10) lorsque la portion de stockage liquide (10) est fixée au corps principal (18), de telle sorte que l'élément capillaire (2) entre en communication fluide avec le substrat formant aérosol liquide (14) stocké dans la portion de stockage liquide (10).

2. Système de génération d'aérosol selon la revendication 1, dans lequel l'élément capillaire (2) a une forme généralement cylindrique avec une première surface au niveau de la première extrémité et une deuxième surface au niveau de la deuxième extrémité, dans lequel l'élément de chauffage (8) est fourni sur la deuxième surface d'extrémité de l'élément capillaire.

3. Système de génération d'aérosol selon la revendication 1 ou la revendication 2, dans lequel l'élément de chauffage (8) est fourni au niveau de la circonférence de l'élément capillaire (2) adjacente à la deuxième extrémité de l'élément capillaire (6).

4. Système de génération d'aérosol selon l'une quelconque des revendications précédentes, dans lequel l'élément de chauffage (8) est un dispositif de chauffage à résistance électrique.

5. Système de génération d'aérosol selon l'une quelconque des revendications précédentes, dans lequel l'élément de chauffage (8) est fourni sous forme d'un revêtement métallique, d'un dispositif de chauffage en treillis ou d'une bobine.

6. Système de génération d'aérosol selon l'une quelconque des revendications précédentes, dans lequel le gradient de taille de pores de l'élément capillaire (2) est un gradient de taille de pores linéaire.

7. Système de génération d'aérosol selon l'une quelconque des revendications précédentes, dans lequel la taille des pores des petits pores (16) est de 4 microns.

8. Système de génération d'aérosol selon l'une quelconque des revendications précédentes, dans lequel la taille des pores des grands pores (12) est entre 10 et 250 microns, de préférence entre 15 et 100 microns, et de manière davantage préférée entre 20 et 50 microns.

9. Système de génération d'aérosol selon l'une quelconque des revendications précédentes, comprenant en outre un élément d'étanchéité devant être disposé entre la circonférence de l'élément capillaire (2) et la portion de stockage de liquide (10) remplaçable, afin d'éviter une fuite non souhaitée du substrat formant aérosol liquide (14) hors de la portion de stockage liquide (10).
